(19) Europäisches Patentamt
European Patent Office
Office européen des brevets

(11) **EP 1 363 933 B1**

(12) **EUROPÄISCHE PATENTSCHRIFT**

(45) Veröffentlichungstag und Bekanntmachung des Hinweises auf die Patenterteilung:
**06.05.2009 Patentblatt 2009/19**

(51) Int Cl.:
*C07J 43/00* (2006.01)      *A61K 9/127* (2006.01)
*A61K 31/58* (2006.01)

(21) Anmeldenummer: 02712923.8

(22) Anmeldetag: **21.02.2002**

(86) Internationale Anmeldenummer:
**PCT/EP2002/001879**

(87) Internationale Veröffentlichungsnummer:
**WO 2002/066490 (29.08.2002 Gazette 2002/35)**

(54) **KATIONISCHE STEROLDERIVATE, PH-SENSITIVE LIPOSOMEN DIESE UMFASSEND UND VERFAHREN ZUR WIRKSTOFFBELADUNG VON LIPOSOMEN**

STEROL DERIVATIVES, LIPOSOMES COMPRISING STEROL DERIVATIVES AND METHOD FOR LOADING LIPOSOMES WITH ACTIVE SUBSTANCES

DERIVES DE STEROLS, LIPOSOMES LES CONTENANT ET PROCEDE DE PERMETTANT DE CHARGER LES LIPOSOMES DE PRINCIPES ACTIFS

(84) Benannte Vertragsstaaten:
**AT BE CH CY DE DK ES FI FR GB GR IE IT LI LU MC NL PT SE TR**

(30) Priorität: **21.02.2001 DE 10109898**

(43) Veröffentlichungstag der Anmeldung:
**26.11.2003 Patentblatt 2003/48**

(73) Patentinhaber: **novosom AG**
**06120 Halle (DE)**

(72) Erfinder:
• **PANZNER, Steffen**
**06114 Halle (DE)**
• **ENDERT, Gerold**
**06114 Halle (DE)**
• **FANKHÄNEL, Stefan**
**04451 Borsdorf (DE)**
• **BEHRENS, Anja**
**50933 Köln (DE)**

(74) Vertreter: **Ziebig, Marlene et al**
**Gulde Hengelhaupt Ziebig & Schneider**
**Patentanwälte Rechtsanwälte**
**Wallstrasse 58/59**
**10179 Berlin (DE)**

(56) Entgegenhaltungen:
WO-A-02/00680      WO-A-97/31935
WO-A-97/39019      US-A- 5 365 487

• BUDKER V ET AL: "PH-SENSITIVE, CATIONIC LIPOSOMES: A NEW SYNTHETIC VIRUS-LIKE VECTOR" BIO/TECHNOLOGY, NATURE PUBLISHING CO. NEW YORK, US, Bd. 14, Juni 1996 (1996-06), Seiten 760-764, XP002937150 ISSN: 0733-222X
• TANG F ET AL: "INTRODUCTION OF A DISULFIDE BOND INTO A CATIONIC LIPID ENHANCES TRANSGENE EXPRESSION OF PLASMID DNA" BIOCHEMICAL AND BIOPHYSICAL RESEARCH COMMUNICATIONS, ACADEMIC PRESS INC. ORLANDO, FL, US, Bd. 242, Nr. 1, 6. Januar 1998 (1998-01-06), Seiten 141-145, XP002072137 ISSN: 0006-291X
• FICHERT T ET AL: "Synthesis and transfection properties of novel non-toxic monocationic lipids. variation of lipid anchor, spacer and head group structure" BIOORGANIC & MEDICINAL CHEMISTRY LETTERS, OXFORD, GB, Bd. 10, Nr. 8, April 2000 (2000-04), Seiten 787-791, XP004203497 ISSN: 0960-894X
• KONSTANTINOVA T V ET AL: "[Synthesis of cholesterol-containing cationic amphiphiles with heterocyclic bases]" RUSSIAN JOURNAL OF BIOORGANIC CHEMISTRY, Bd. 27, Nr. 6, November 2001 (2001-11), Seiten 404-407, XP002207246 ISSN: 0132-3423

Bemerkungen:
Die Akte enthält technische Angaben, die nach dem Eingang der Anmeldung eingereicht wurden und die nicht in dieser Patentschrift enthalten sind.

**EP 1 363 933 B1**

## Beschreibung

Die Erfindung betrifft polare Verbindungen auf Basis eines Sterolgerüstes, wobei an die 3-Position des Ringsystems ein organisches Kation mit einem pK-Wert zwischen 3,5 und 8 substituiert ist. Die Erfindung betrifft auch Liposomen, die diese Verbindungen enthalten.

Unter dem Begriff der Lipide werden drei Klassen von Naturstoffen zusammengefasst, die sich aus biologischen Membranen isolieren lassen: Phospholipide, Sphingolipide und Cholesterol mit den Derivaten.

Von technischem Interesse sind diese Substanzen bei der Herstellung von Liposomen. Diese Liposomen lassen sich unter anderem als Container für Wirkstoffe bei pharmazeutischen Zubereitungen einsetzen. Wünschenswert ist dabei eine effiziente und stabile Verpackung des Cargos und eine kontrollierbare Freisetzung des Inhalts. Beide Ansprüche sind nicht ohne weiteres zu vereinen: Je stabiler und dichter die Verpackung ist, desto schwerer gibt sie den eingeschlossenen Wirkstoff wieder frei. Aus diesem Grund wurden Liposomen entwickelt, die ihre Eigenschaften als Reaktion auf einen äußeren Reiz verändern. Bekannt sind thermosensible und pH-sensitive Liposomen. Die pH-sensitiven Liposomen sind von besonderem Interesse, da dieser Parameter sich auch unter physiologischen Umständen, etwa bei der endozytotischen Aufnahme eines Liposoms in Zellen oder bei der Passage des Magen-Darm-Trakts, ändern kann.

Im weiteren sollen folgende Abkürzungen verwendet werden:

CHEMS     Cholesterolhemisuccinat
PC        Phospatidylcholin
PE        Phosphatidylethanolamin
PS        Phosphatidylserin
His-Chol  Histaminylethanamin-Cholesterolhemisuccinat
Py-Chol   Pyridylethanamin-Cholesterolhemisuccinat
Mo-Chol   Morpholinoethanamin-Cholesterolhemisuccinat

Nach dem Stand der Technik umfassen pH-sensitive Liposomen insbesondere CHEMS. CHEMS in Mischung mit Phosphatidylethanolamin zur Herstellung pH-sensitiver Liposomen verwendet (Tachibana et al.(1998); BBRC 251: 538-544, US4891208). Nature Biotechnology Band 14, S.760-7 (1996) beschreibt pH-sensitive Liposomen, die die Verbindungen ChIm enthalten. Solche Liposomen können von Zellen endozytiert werden und vermögen auf diesem Weg Cargomoleküle in das Innere von Zellen zu transportieren, ohne die Integrität der zellulären Membran zu verletzen.

Ein wesentlicher Nachteil des CHEMS ist dessen anionischer Charakter. Die damit hergestellten Liposomen besitzen eine negative Gesamtladung und werden nachteiliger weise nur mit geringer Effizienz von Zellen aufgenommen. Trotz des oben beschriebenen Transfermechanismus eignen sie sich daher kaum für den Eintransport von Makromolekülen in Zellen:

Für diesen Zweck werden fachgemäß kationische Liposomen verwendet, die über eine möglichst hohe und konstante berflächenladung verfügen. Die positive Gesamtladung solcher Partikel führt zu einer elektrostatischen Anheftung an Zellen und in der Folge zu einem effizienten Eintransport. Der Einsatz dieser Verbindungen und der damit hergestellten Liposomen bleibt aber auf Anwendungen in vitro oder ex vivo beschränkt, da solche positiv geladenen Liposomen mit Serumbestandteilen nachteilig unkontrollierte Aggregate bilden.

Es bestand daher die Aufgabe, neue Verbindungen herzustellen,

i) mit deren Hilfe Wirkstoffe in Liposomen eingeschlossen und bei einer Veränderung des pH-Wertes wieder freigesetzt werden können und

ii) durch deren Anwesenheit die Herstellung von kationischen Liposomen gelingt, die ohne Bildung von größeren Aggregaten mit Serum gemischt werden können.

Weitere Aufgaben der Erfindung bestanden darin, dass die gesuchten Verbindungen sich einfach und preiswert herstellen lassen und in hohem Anteil in liposomale Membranen eingebaut werden können.

Die erfindungsgemäße Aufgabe wird gelöst durch ein Sterolderivat mit einem pKa-Wert zwischen 3,5 und 8 nach der allgemeinen Formel:

$$\text{Kation} - \text{Spacer 2} - Y - \text{Spacer 1} - X - \text{Sterol,}$$

wobei Y und X verbindende Gruppe darstellen. Je nach verwendetem Kation erhält man Verbindungen, die bei einem spezifischen pH-Wert ihre Ladung ändern und sich durch die Sterolkomponente zu einem hohen Anteil in liposomale Membranen einbauen lassen. Als Ausgangsverbindung können einfache und billige Sterole oder deren Derivate verwendet werden. Demgemäß kann die erfindungsgemäße Aufgabe durch Konjugation von pH-sensitiven Kationen an die 3-Position eines Sterolgerüstes gelöst werden.

Unter den membranbildenden oder membranständigen Gruppen einer biologischen Bilayermembran sind die Sterole von besonderem Interesse, da diese Verbindungen billig verfügbar sind, eine einfache Chemie aufweisen und in einem hohen Anteil in Membranen eingebaut werden können, ohne deren Permeabilität zu erhöhen oder gar den Membrancharakter völlig zu zerstören. Wichtig für den Erhalt der letztgenannten Charakteristik ist jedoch, dass die Substitution mit einem polaren Molekül an der 3-Position des Sterols erfolgt.

Das Sterol ist beispielsweise Cholesterol. Zwischen der kationischen Gruppe und dem Sterolgerüst liegen die Molekülfragmente Spacer 2 - Y - Spacer 1 - X..

Das Gesamtmolekül erhält seine pH-abhängige Ladungscharakteristik durch ein oder mehrere organische Kationen mit einem pKa-Wert zwischen 3,5 und 8. Typische Moleküle oder Molekülfragmente mit dieser Eigenschaft sind Stickstoffbasen. Diese Stickstoffbasen werden über Spacer und Kopplungsgruppen mit der 3-Position des Sterolgerüsts verknüpft, so dass eine Verbindung nach der erfindungsgemäßen Formel entsteht.

Da die Stickstoffbasen als Ringsysteme vorliegen, existieren Stellungsisomere, bei denen der verbindende Spacer an verschiedenen Positionen des organischen Kations substituiert ist. Solche Stellungsisomere fallen unter den Offenbarungsgehalt dieser Erfindung. Durch die Stellungsisomerie allein lassen sich in vielen Fällen bereits die pKa-Werte der organischen Kationen beeinflussen. Die dem zugrunde liegenden Regeln sind dem Fachmann geläufig. Alternativ können diese Einflüsse aus Tabellenwerken abgeschätzt werden (Handbook of Chemistry and Physics, 73. Band, S. 8-37 ff.).

In einer bevorzugten Ausführungsvariante der Erfindung weist das Sterolderivat einen pKa-Wert zwischen 4 und 6,5 auf. Dieser pKa-Wert liegt vorteilhafter Weise in einem Bereich, der für die Physiologie zahlreicher Organismen eine entscheidende Bedeutung besitzt.

Die Kationen sind Stickstoffbasen ausgewählt aus Piperazine, Imidazole, Morpholine, Purine Pyrimidine und 2-, 3- oder 4-modifizierte Pyridine.

Durch Ankopplungsreaktionen entstehen amphiphile organische Kationen, die beispielsweise aus den folgenden Stoffklassen stammen:

2-, 3-, 5-,6-, 7- oder 8-substituierte Benzimidazole, 2-, 3, 4- oder 5-substituierte Imidazole, 2-,3- oder 4- substituierte Morpholine, 1-, 2-,oder 3- substituierte Piperazine, 3-,4-, 5-, 6- oder 9- substituierte Purine, 2-, 3- oder 4- modifizierte Pyridine, 2- , 4-, 5- oder 6-subsituierte Pyrimidine.

Ganz besonders bevorzugt sind solche Molekülfragmente, wie sie in biologischen Systemen vorkommen, also beispielsweise 4-Imidazole (Histamine), 2-,6- oder 9- Purine (Adenine, Guanine, Adenosine oder Guanosine), 1-, 2- oder 4-Pyrimidine (Uracile, Thymine, Cytosine, Uridine, Thymidine, Cytidine) oder auch Pyridin-3-carbonsäuren (Nicotinsäureester oder - amide).

Die hier genannten Strukturfragmente können weitere Substituenten aufweisen. Das können beispielsweise Methyl-, Ethyl-, Propyl- oder Isopropylreste sein, besonders bevorzugt in hydroxylierte Form mit einer oder zwei Hydroxylgruppen. Das können aber auch Hydroxyl- oder Ketofunktionen des Ringsystems sein. Daneben sind weitere Strukturfragmente möglich, insofern sie im pH-Bereich zwischen 3,5 und 8,5 keine anionisch dissoziierten Molekülteile bilden, wie beispielsweise Carbonsäuren, Sulfonsäuren oder manche aromatische Hydroxylgruppen oder Enole.

Anionische Gruppen wie etwa Carbonsäuren, Sulfonsäuren, Enole oder aromatische Hydroxyle dürfen nur dann Bestandteil des Moleküls sein, wenn sie im beanspruchten pH-Bereich zwischen 3,5 und 8,5 nicht dissoziiert vorliegen. Das ist im allgemeine der Fall, wenn deren pKa-Wert oberhalb von 9,5 liegt.

Die verbindenden Gruppen X und Y sind ausgewählt aus der Gruppe bestehend aus -(C=O)-O-; -(C=O)-NH-; -(C=O)-S-; -O-; -NH-; -S- -CH=N- O-(O=C)-; -S-(O=C)-; -NH-(O=C)- und -N=CH-.

Spacer 1 ist ein Niederalkylrest mit linearer, verzweigter oder ringförmiger Struktur, der bis zu 8 C-Atome besitzt und 0, 1 oder 2 ethylenische ungesättigte Bindungen enthält. Spacer 1 kann zur Erhöhung der Polarität des Moleküls Hydroxylgruppen besitzen. Spacer 1 kann insbesondere ein Zucker sein. Spacer 2 ist ein Niederalkylrest mit linearer, verzweigter oder ringförmiger Struktur, der bis zu 8 C-Atome besitzt und 0, 1 oder 2 ethylenische ungesättigte Bindungen enthält. Spacer 2 kann zur Erhöhung der Polarität des Moleküls Hydroxylgruppen besitzen. Spacer 2 kann insbesondere ein Zucker sein.

Methoden zur Ausführung solcher Kopplungsreaktionen sind dem Fachmann bekannt und werden je nach verwendetem Ausgangsstoff und Kopplungskomponente variieren. Typische Reaktionen sind die Veresterung, die Amidierung, die Addition von Aminen an Doppelbindungen, die Veretherung oder auch die reduktive Aminierung.

Eine besonders bevorzugte Methode der Kopplung ist die Amidierung von Hemisuccinaten der Sterole. Moleküle, die die Anforderungen der erfinderischen Aufgabe befriedigen, lassen sich unter anderem durch Kopplung von Histamin, N-(2-Aminoethyl)-Morpholin oder N-(2-Aminoethyl)-piperazin herstellen. Die so erhaltenen Verbindungen werden hier als His-Chol, Mo-Chol oder Pip-Chol bezeichnet.

Das Sterol ist ausgewählt aus der Gruppe bestehend aus: Cholesterol, das Sitosterol, das Campesterol, das Desmosterol, das Fucosterol, das 22-Ketosterol, das 20-Hydroxysterol, das Stigmasterol, das 22-Hydroxycholesterol, das 25-Hydroxycholesterol, das Lanosterol, das 7-Dehydrocholesterol, das Dihydrocholesterol, das 19-Hydroxycholesterol, das 5α-Cholest-7-en-3β-ol, das 7-Hydroxycholesterol, das Epicholesterol, das Ergosterol und das Dehydroergosterol.

Die verwendeten Sterole können an ihrer 3-Position verschiedene Gruppen tragen, die eine einfache und beständige Ankopplung erlauben oder optional die Funktion eines räumlichen Spacers erfüllen. Besonders geeignet für eine direkte Kopplung sind die natürlicherweise vorhandene Hydroxylgruppe, aber auch das Chlor der Sterolchloride, oder etwa die Aminogruppe der Sterolamine oder die Thiolgruppe des Thiocholesterols.

Die Erfindung betrifft auch Liposomen, die die erfinderischen Substanzen umfassen. Alle erfindungsgemäßen Substanzen oder Verbindungen lassen sich in einem hohen Anteil in liposomale Membranen einbauen und führen erst dann zu einer positiven Ladung des Gesamtpartikels, wenn der pH-Wert des Mediums kleiner als (pKa+1) der erfindungsgemäßen Verbindungen ist.

In einer besonderen Ausführungsvariante der Erfindung beträgt der Anteil des Sterolderivats maximal 50 mol%. Besonders bevorzugt sind Zusammensetzungen, die mindestens 5 mol%, höchstens aber 40 mol% der Verbindung enthalten. Ganz besonders bevorzugt sind Zusammensetzungen, die mindestens 10 mol% und höchsten 30 mol% des Sterolderivats enthalten.

Zweckmäßig ist weiterhin eine Ausführungsvariante, bei der die Liposomen insbesondere Phosphatidylcholin, Phophatidyletanolamin und/oder Diacylglycerol umfassen. Da Cholesterole selbst keine Liposomen ausbilden können, ist der Zusatz eines weiteren Lipids notwendig. Dieses Lipid kann insbesondere ein Phospholipid sein. Weitere Modifikationen des Liposoms sind selbstverständlich möglich. So ist insbesondere die Verwendung Polyethylenglykol-modifizierter Phospholipide oder analoger Verbindungen vorteilhaft.

In einer weiteren Ausführungsvariante der Erfindung weisen die Liposomen eine mittlere Größe zwischen 50 und 1000 nm, bevorzugt zwischen 50 und 300 nm und ganz besonders bevorzugt zwischen 60 und 130 nm auf.

In einer weiteren bevorzugten Ausführungsvariante umfassen die Liposomen Wirkstoffe. Die erfindungsgemäßen Liposomen sind beispielsweise für die parenterale Anwendung geeignet. Sie können z.B. in der Krebstherapie und zur Therapie schwerer Infektionen verwendet werden. Liposomendispersionen können dazu injiziert, infundiert oder implantiert werden. Sie verteilen sich danach im Blut oder in der Lymphe oder geben als Depot ihren Wirkstoff kontrolliert ab. Letzteres kann durch hochkonzentrierte Dispersionen erreicht werden, die als Gele vorliegen. Die Liposomen können auch für topische Anwendung auf der Haut eingesetzt werden. Sie können insbesondere dazu beitragen, dass verschiedene Wirkstoffe besser in die Haut eindringen können oder sogar durch die Haut in den Körper gelangen können. Weiterhin ist es möglich die Liposomen für den Gentransfer einzusetzen. Genetisches Material kann wegen seiner Größe und Ladung meist nicht ohne Hilfsmittel in Zellen gelangen. Dazu bedarf es geeigneter Träger wie z.B. Liposomen oder Lipid-Komplexen. Diese sollen zusammen mit der DNA effizient und möglichst gezielt in die betroffenen Zellen aufgenommen werden. Dazu werden zelleigene Transportmechanismen wie die Endozytose herangezogen. Die erfindungsgemäßen Liposomen sind selbsverständlich auch als Modellmembranen einsetzbar. Liposomen sind in ihrer prinzipiellen Struktur den Zellmembranen sehr ähnlich. Sie können daher als Membranmodelle eingesetzt werden, um die Permeationsgeschwindigkeit von Wirkstoffen durch Membranen oder die Membranbindung von Wirkstoffen zu quantifizieren.

Liposomen, die unter Verwendung der erfindungsgemäßen Substanzen hergestellt werden, zeigen vorteilhafter Weise nur eine geringe unspezifische Bindung an Zelloberflächen. Diese geringe unspezifische Bindung ist eine wesentliche Voraussetzung für das Zustandekommen einer spezifischen Bindung an Zielzellen. Werden die beschriebenen Liposomen mit weiteren Liganden versehen, so ist eine Zielsteuerung der Vehikel gegeben. Der Wirkstoff kann dann spezifisch an solchen Zellen oder Geweben angereichert werden, die pathologische Zustände aufweisen.

Eine wesentliche Verwendung der erfindungsgemäßen Substanzen liegt daher bei der Konstruktion von Vektoren für den Wirkstofftransfer in lebenden Organismen. Die Vektoren sind besonders geeignet für den Transport von therapeutischen Makromolekülen wie etwa Proteinen oder DNA, die von sich aus nicht die Zellmembran überwinden können oder im Blutstrom schnell abgebaut werden.

Mit Vorteil können Antikörper, Lektine, Hormone oder andere Wirkstoffe unter schonenden Bedingungen in hoher Ausbeute auf die Oberfläche von Liposomen gekoppelt werden. In einer Variante der erfindungsgemäßen Lehre umfassen Liposomen für diese Verwendung eine hinreichende Menge an PDEA-Chol neben anderen Lipiden, auch solchen, wie sie in der vorliegenden Beschreibung erwähnt werden. Die Menge des verwendeten PDEA-Chol richtet sich dabei nach gewünschten Verwendungszweck. So brauchen Liposomen, die mit einem Marker beladen sind, ein hohes Verhältnis von diesem Signalgeber zur spezifitätsbestimmenden Komponente. In diesem Fall ist nur eine geringe Anzahl von Antikörper pro Liposom zu koppeln.

In einer bevorzugten Ausführungsvariante der Erfindung umfassen die Liposomen als Wirkstoff ein Protein, ein Peptid, eine DNA, eine RNA, ein antisense-Nukleotid und/oder ein Decoy-Nukleotid.

In einer besonders bevorzugten Ausführungsvariante befinden sich mindestens 80% des Wirkstoffes im Innern des Liposoms.

Die Erfindung betrifft auch ein Verfahren zur Wirkstoffbeladung der Liposomen, wobei ein definierter pH-Wert zur Verkapselung benutzt wird und ein zweiter pH-Wert zur Abtrennung des nicht gebundenen Wirkstoffes eingestellt wird.

Die Erfindung betrifft auch ein Verfahren zur Wirkstoffbeladung der Liposomen wobei, die Liposomen bei einem definierten pH-Wert permeabilisiert und verschlossen werden.

Die Erfindung betrifft auch Verwendung der Liopsomen zur Herstellung von Nanokapseln.

Die Erfindung betrifft weiterhin Verwendung der Liposomen zur Herstellung von Freisetzungssystemen in der Diagnostik.

Mit Vorteil werden die Liposomen zum Transport und/oder zur Freisetzung von Wirkstoffen verwendet.

Zweckmäßig ist in einer weiteren Ausgestaltung Verwendung der Liposomen Depotformulierung und/oder als zirkulierendes Depot.

Mit Vorteil können die Liposomen insbesondere bei intravenöser oder peritonealer Applikation verwendet werden.

Vorteilhaft ist in einer weiteren Ausgestaltung der Erfindung die Verwendung der Liposomen als Vektor zur Transfektion von Zellen in vitro und ex vivo. Ebenso vorteilhaft sind die Liposomen zur Verwendung als Vektor zur Transfektion in vivo.

Überraschenderweise konnte festgestellt werden, dass die Permeabilität der Lipidschicht der erfindungsgemäßen Liposomen insbesondere vom pH-Wert und damit vom Ladungszustand des Sterolderivats abhängig ist. Bei der Verwendung des bekannten CHEMS tritt eine Permeabilitäterhöhung zudem nur bei der gleichzeitigen Anwesenheit hoher Mengen von Phosphatidylethanolamin (PE) in der Membran auf. Dieses Phospholipid bildet für sich allein keine Membranen und wird durch das CHEMS künstlich stabilisiert. Nachteilig bei solchen Liposomen ist aber deren geringe Stabilität, die sich darin äußert, dass kleinere Wirkstoffmoleküle auch ohne pH-Wechsel bereits langsam ausdiffundieren.

Wird insbesondere His-Chol verwendet, so werden auch Membranen aus Phosphatidylcholin (PC) so permeabilisiert, dass eingeschlossene Wirkstoffe oder Marker innerhalb von Minuten bis Stunden ausdiffundieren. Diese Membranen sind für sich genommen jedoch stabil und zeigen nur eine geringe Anfangspermeabilität. Liposomen unter Verwendung der erfindungsgemäßen Strukturen sind daher geeignet zur Konstruktion von Freisetzungssystemen, bei denen eine Abgabe von Wirkstoffen in Abhängigkeit vom pH-Wert des Mediums erfolgen soll.

Es wurde darüber hinaus überraschenderweise gefunden, das in Liposomen, deren Membran die hier beschriebenen Verbindungen enthält, überdurchschnittlich hohe Mengen an Proteinen oder DNA eingeschlossen werden können. Die Effizienz dieses Einbaus ist dabei abhängig vom pH-Wert der verwendeten Lösung. Ein Prozess für die effiziente Verkapselung von Proteinen oder DNA in die Liposomen kann daher so geführt werden, dass zunächst ein pH-Wert eingestellt wird, der zu einer guten Bindung der Cargomoleküle in die Liposomen führt. Für DNA als Polyanion werden hier niedrige pH-Werte von etwa 4 bis 5 verwendet. Bei Proteinen richtet sich der nutzbare pH-Wert nach dem isoelektrischen Punkt des Proteins. Dieser sollte unterhalb des pKa-Wertes der erfindungsgemäßen Substanz liegen. Eine Verkapselung ist dann besonders effektiv, wenn der pH-Wert des Mediums so gewählt wird, dass er zwischen dem isoelektrischen Punkt des Proteins und dem pKa-Wert des Sterolderivates liegt. Die Proteine sind dann negativ geladen, die Lipidschicht hat bereits eine positive Nettoladung.

Nicht eingebaute, außen anhaftende Cargomoleküle können wenn nötig durch einen einfache Erhöhung des pH-Wertes entfernt werden. Dieser Schritt ist immer dann notwendig, wenn nicht eingebaute Cargomoleküle zu einer Aggregation der Liposomen führen. Vorteilhaft bei der Verwendung der erfindungsgemäßen Komponenten ist die Tatsache, dass die eingeschlossenen Wirkstoffe nur für den Zeitraum des eigentlichen Einschlusses unter Bedingungen gebracht werden müssen, die eine Interaktion mit der Lipidschicht erlauben. Sobald die Lipidschicht in sich geschlossen bleibt, kann zu anderen Bedingungen gewechselt werden. Eine denkbare Inaktivierung von Wirkstoffen, insbesondere von Proteinen kann dadurch minimiert werden.

Liposomen, die die erfindungsgemäßen Komponenten umfassen, können unter dem Fachmann bekannten Bedingungen mit Polymeren beschichtet werden. Dabei kann insbesondere eine einfache oder mehrfache Abscheidung solcher Substanzen auf der Oberfläche erfolgen. Bei einer mehrfachen Abscheidung, die gegebenenfalls unter Anwesenheit von Vernetzer durchgeführt wird, entstehen liposomale Nanoakapseln, wie sie in der WO 00/28972 oder in der WO01/64330 beschrieben sind.

Vorteilhaft bei der Verwendung der hier beschriebenen Substanzen ist die Tatsache, dass die elektrostatische Interaktion mit dem Polyelektrolyten unterbrochen werden kann. Es ist bekannt, dass die Wechselwirkung eines Polyelektrolyten mit Ladungsträgern der liposomalen Membran zur Entmischung von Membranbestandteilen und zur Bildung von Lipidclustern führen kann. In vielen Fällen geht diese Entmischung mit einer Permeabilisierung des Liposoms einer. Die erfindungsgemäßen Substanzen ermöglichen eine Abschaltung dieser Wechselwirkung nach dem Beschichtungsprozess. Wird der pH-Wert zu diesem Zeitpunkt erhöht, so sind die Liposomen nur noch sterisch in der Nanokapseln eingeschlossen, eine Wechselwirkung der Membran mit den Polyelektrolyten besteht dann nicht mehr. Clusterbildung der Lipide und damit verbundene Permeabilisierung der Membran können so umgangen werden.

In einer Variante dieser erfindungsgemäßen Lehre werden die Permeabilitätsänderungen gezielt zur Beladung von Liposomen genutzt. Ein einzuschließender Wirkstoff kann dabei unter Bedingungen hoher Permeabilität ins Medium zugegeben werden. Anschließend werden Bedingungen geringer Permeabilität eingestellt. Damit verbleibt der Wirkstoff im Innern der Liposomen. Nicht eingeschlossener Wirkstoff kann dann gegebenenfalls abgetrennt werden. Eine solche Permeabilitätsänderung kann an Liposomen oder an liposomalen Nanokapseln herbeigeführt werden.

Weiterhin wurde überraschend gefunden, dass Liposomen, deren Membran His-Chol oder Pip-Chol enthält, zur Chelatisierung von Metallionen befähigt sind. Diese Eigenschaft führt zu einer Verstärkung der positiven Ladung des Liposoms. Dieser Effekt ist besonders stark bei neutralen pH-Werten zu beobachten, da dann die Eigenladung der Verbindung gering ist. Aufgrund ihrer chelatisierenden Eigenschaften lassen sich solche Liposomen in der biochemischen Diagnostik und zur pharmazeutischen Therapie verwenden.

In einem Detektionssystem können solche Liposomen mit Metallionen beladen werden, deren Fluoreszens durch die Chelatisierung verstärkt wird, also beispielsweise Terbium- oder Europiumionen. Liposomen für diese Anwendung enthalten darüber hinaus spezifitätsbestimmende Komponenten, also z.B. Antikörper, Lektine, Selektine, Rezeptoren oder Hormone oder RNA-Aptamere. In einer besonders bevorzugten Ausführungsform der erfindungsgemäßen Verwendung ist die Anwesenheit dieser Metallionen auf das Lumen der Liposomen beschränkt, um unspezifische Signale von außen anhaftenden und langsam freigesetzten Metallionen zu vermeiden.

Weiterhin wurde überraschend gefunden, dass die erfindungsgemäßen Liposomen bei niedrigem pH-Wert leicht mit anderen Membranen fusionieren. Für gewöhnlich erfordert dieser Schritt die Anwesenheit eines größeren Anteils von PE in der Membran. Dieses hat durch seine Neigung zur Bildung von hexgonalen Phasen die Funktion eines Helferlipids. Nachteilig ist aber die geringere Stabilität solcher Membranen, hier wird oft eine schleichende Freisetzung von eingeschlossenen Wirkstoffen beobachtet.

Liposomen, die unter Verwendung der erfindungsgemäßen Substanzen hergestellt werden, fusionieren aber effektiv auch in Abwesenheit eines solchen Helferlipids. Es sind also unter Verwendung der erfindungsgemäßen Substanzen solche Liposomen herstellbar, die einen Wirkstoff stabil verkapseln können, ober unter den Bedingungen eines niedrigen pH-Werts mit Zellmembranen fusionieren und dort den Wirkstoff freisetzen.

Diese Kombination zweier Eigenschaften stellt eine wichtige Voraussetzung für die Einbringung von Cargomolekülen in Zellen dar. Bei einer Fusion von Liposomen mit Zellhüllen oder -bestandteilen vereinigen sich die wässrigen Lumen beider Partner, ohne das es zu einer Öffnung der Membranstrukturen gegenüber dem Medium kommt. Dadurch wird der unkontrollierte Ein- oder Ausstrom anderer Substanzen vermieden.

Eine wesentliche Voraussetzung für die Verwendung von Liposomen für experimentelle oder therapeutische Zwecke ist deren Verträglichkeit mit Zellen und Geweben. Eine Reihe bekannter Verbindungen, die für das Einbringen von DNA oder Proteinen in Zellen genutzt werden (beispielsweise das kationische Lipid DOTAP) sind zytotoxisch.

Es wurde weiterhin überraschenderweise gefunden, dass die erfindungsgemäßen Verbindungen eine verringerte Zytotoxizität zeigen. Solche Messungen sind im Beispielteil wiedergegeben. So zeigt His-Chol im MTT-Test eine geringere toxische Wirkungen im Vergleich zum vielfach angewendeten DOTAP.

Eine weitere Voraussetzung für die Konstruktion von Vektoren zum Gen- oder Proteintransport in Zellen ist deren Kompatibilität mit Serum oder Blut. Wegen ihrer starken kationischen Ladung bilden die derzeit bekannten Vektoren mit Serum unkontrollierte große Aggregate, die im Organismus zur Bildung von Thromben führen. Ihre Verwendung ist damit in vivo praktisch ausgeschlossen und auf in vitro oder ex vivo Anwendungen beschränkt.

Es wurde überraschenderweise gefunden, dass Liposomen, die unter Verwendung der erfindungsgemäßen Komponenten aufgebaut wurden, in Serum oder Blut keine Aggregate bilden.

Eine nächste Voraussetzung für die Konstruktion von Vektoren zum Protein- oder Gentransfer ist deren Stabilität unter physiologischen Bedingungen. Liposomen werden bei einer Applikation in den Blutkreislauf vom Bestandteilen des Complementsystems angegriffen und schnell lysiert. Diese Reaktion erfolgt binnen Minuten. Es kommt zur Porenbildung in der Membran, durch die selbst große Moleküle wie Proteine ausdiffundieren können. Eine Stabilisierung von Liposomen gegenüber diesen Mechanismen ist bisher nur durch Einbau von Cholesterol in die Lipidschicht möglich. Solche Liposomen sind dann sehr stabil, können aber nicht mehr mit Zellen wechselwirken oder ihren Wirkstoff leicht abgeben. Es wurde überraschenderweise gefunden, dass Liposomen, die unter Verwendung der erfindungsgemäßen Komponenten aufgebaut werden, in Serum oder Blut über mehrere Stunden stabil sind. Die Wirkstofffreisetzung ist auch unter diesen Bedingungen gering.

Eine liposomaler Vektor für den Transport von Wirkstoffen muss mindestens drei Voraussetzungen erfüllen: er muss eine geringe Toxizität besitzen, den Wirkstoff sicher und stabil einschließen und kompatibel mit Serum oder Blut sein.

Alle drei dieser Voraussetzungen werden von Liposomen, die unter Verwendung der erfindungsgemäßen Substanzen hergestellt sind, erfüllt. Die hier offenbarten Liposomen sind daher für eine Verwendung bei therapeutischen Zwecken gut geeignet. Weitere Eigenschaften, die diese Verwendung unterstützen sind die gute Beladbarkeit mit Wirkstoffen und die gezielte Freisetzung dieser Stoffe durch Permeabilisierung der Membran bei geeigneten pH-Werten oder Redoxzuständen.

Die Erfindung soll im folgenden anhand von Beispielen näher erläutert werden, ohne dass die Erfindung auf diese Beispiele zu beschränken ist.

**Beispiel 1**

Synthese von His-Chol

1.31 g Cholesterylhemisuccinat werden bei Raumtemperatur in 20 ml DMF gelöst. Zur Lösung werden 438 mg Carbonyldiimidazol, gelöst in 20 ml DMF gegeben. Man lässt das Gemisch 1 Stunde rühren und gibt dann 300 mg Histamin zu. Nachdem das Gemisch über Nacht gerührt wurde, engt man im Vakuum vollständig ein. Der Rückstand wird säulenchromatografisch an Kieselgel 60 gereinigt, als Laufmittel wird Chloroform/Methanol 10:1 verwendet.

(Ausbeute 54%, 1.45 mol), sauber in der HPLC, Identität bestimmt per MS und 13C-NMR.

**Vergleichsbeispiel 2**

Synthese von PDEA-Chol

Bei der Synthese von PDEA-Chol verfährt man wie oben. Anstelle des Histamins werden 600 mg Pyridyldithioethanaminhydrochlorid verwendet.

**Beispiel 3**

Synthese von Mo-CHol

Bei der Synthese von Mo-Chol verfährt man wie oben. Anstelle des Histamins werden 350 mg 4-(2-Aminoethyl)-morpholin verwendet.

**Beispiel 4**

Herstellung von kationischen pH-sensitiven Liposomen

5 mg His-Chol und 9.8 mg POPC werden in 4 ml Chloroform/Methanol (1:1, v/v) gelöst und im Rotationsverdampfer vollständig getrocknet. Der Lipidfilm wird mit 4.3 ml des entsprechenden Puffers (10 mM KAc, 10 mM HEPES, 150 mM NaCl, pH 7.5) in einer Lipidkonzentration von 5mM durch kurzes Ultrabeschallen hydratisiert (5 min). Abschließend wird die Emulsion eingefroren und nach dem Tauen mehrfach extrudiert (Avestin LiposoFast, Polycarbonatfilter 200 nm Porenweite).

Der Verlauf des Zetapotentials bei verschiedenen pH-Werten ist in der untenstehenden Tabelle dargestellt.

| pH-Wert | Zetapotential in mV |
|---------|---------------------|
| 4,4     | +52                 |
| 6,2     | -3                  |
| 7,5     | -13                 |

**Beispiel 5**

Permeabilität

Liposomen werden generell wie in Beispiel 4 hergestellt. Folgende Lipidmischungen wurden verwendet (Angaben in Mol-%):

| | | |
|---|---|---|
| A: | DPPC 60 | His Chol 40 |
| B: | DPPC 60 | CHEMS 40 |
| C: | POPC 60 | His Chol 40 |
| D: | POPC 60 | CHEMS 40 |

Die Lipide werden im angegebenen Lösungsmittelgemisch gelöst und unter Vakuum getrocknet. Die Lipidfilme werden mit 100mM Carboxyfluorescein, 50 mM NaCl, pH 7.5 bei einer Lipidkonzentration von 15 mM hydratisiert und wie oben gefroren, getaut und extrudiert. Nicht eingeschlossenes Carboxyfluorescein wird durch Gelfiltration entfernt.

20 μl der so erhaltenen Liposomen werden mit 2 ml Puffer (10 mM Kaliumacetat, 10 mM HEPES) inkubiert. Nach 90 min wird die Menge des ausgetretenen Carboxyfluoresceins durch Messung der Fluoreszensintensität der Probe bestimmt. Eine Vergleichsprobe mit vollständiger Freisetzung des eingeschlossenen Markers wird durch Zugabe von 0,2% Triton X100 zum Ansatz erhalten.

|       | A | B  | C  | D  |
|-------|---|----|----|----|
| pH4,4 | 8 | 38 | 80 | 39 |
| pH5,4 | 7 | 7  | 35 | 18 |
| pH6,4 | 6 | 9  | 18 | 16 |
| pH7,4 | 7 | 7  | 17 | 15 |
| pH8,4 | 5 | 11 | 15 | 15 |

## Beispiel 6

Chelatisierung von Metallionen

Liposomen werden wie in Beispiel 4 hergestellt. 40 μl dieser Liposomen werden in 7 ml Puffer (10 mM Kaliumacetat, 10 mM HEPES pH 4.2 oder pH 7.5) suspendiert. Anschließend werden die Metallionen in den angegeben Konzentrationen zugegeben und das Zetapotential der Liposomen gemessen.

| Ionen |       | pH 4.2  | pH 7.5        |
|-------|-------|---------|---------------|
| $Ni^{2+}$ | 10 mM | + 16.8 | + 11.6   |
| 10 mM | 10 mM | + 40.5  | +4.6          |
| 10 mM | 10 mM | + 65.4  | nicht messbar |
| Ohne Zusatz | | + 47.6 | +2.4         |

Bei neutralem pH ist eine Chelatisierung von Nickelionen deutlich nachweisbar. Bei einem leicht sauren pH werden Nickelionen nicht länger gebunden, wohl aber Zinkionen. Das Nichtübergangsmetall Calcium verhält sich indifferent und bildet keine Chelatkomplexe.

## Beispiel 7

Bindung von DNA

1 mg DNA (Hering sperm, SIGMA D3159) wird in 1ml Wasser gelöst. Von den Liposomen aus Beispiel 4 wird eine 0.2 mM Suspension in Puffer (10 mM Kaliumacetat, 10 mM HEPES, pH 4.2 oder pH 7.5) hergestellt. 45 μl der DNA-Lösung werden zu jeweils 1ml der unterschiedlichen Liposomenproben gegeben und schnell gemischt. Nach 15 min Inkubation wird die Probe mit 6ml des entsprechenden Puffers aufgefüllt und das Zetapotential der Liposomen vermessen.

Zetapotential in mV

|            | ohne DNA | Mit DNA |
|------------|----------|---------|
| pH = 4.2   | + 27.1   | - 45.7  |
| pH = 7.5   | - 6.3    | - 39.6  |

**Beispiel 8**

Fusionseigenschaften

Liposomen mit folgenden Zusammensetzungen werden wie in Beispiel 1 hergestellt (alle Angaben in Mol-%):

| | | | |
|---|---|---|---|
| A) | POPC 60 | HisChol 40 | |
| X) | POPC 100 | | |
| Y) | POPC 60 | DPPG 40 | |

Die fakulativ kationischen Liposomen A werden mit den neutralen Liposomen X oder den anionischen Liposomen Y im Puffer (10 mM HEPES, 10 mM Kaliumacetat, pH 4.2 bzw. 7.5) inkubiert. Die eventuelle Fusion von Liposomen wird mittels Größenmessung durch dynamische Lichtstreuung analysiert.

| | | |
|---|---|---|
| Liposom 1 | X | Y |
| Liposom 2 | A | A |
| pH 4.2 | 181,6 nm | 1689,3 nm |
| pH 7.5 | 191,8 nm | 250,0 nm |

Die Ausgangsgrößen der Liposomen betrugen bei pH 4.2 161,8 nm und 165,9 nm bei pH 7.5

X) 199,2nm
Y) 183, 2nm

Die Größe des komplementär geladenen Paares YA unterscheidet sich deutlich von der Größe der Mischsuspensionen mit dem Neutralliposom XA. Das Ausmaß der Wechselwirkung ist durch das Maß der Aufladung der fakultativ kationischen Liposomen bestimmt. Eine Fusion zu größeren Einheiten ist nicht von dem fusogenen Lipid PE abhängig.

**Beispiel 9**

Permeabilität gegenüber Makromolekülen

15 $\mu$mol DOPE und 10 $\mu$mol HisChol werden in Isopropanol gelöst und das Lösungsmittel wird unter Vakuum abgezogen. Zu dem getrockneten Lipidfilm gibt man 2.5 ml einer Lösung von Proteinase K in Puffer (1 mg/ml Proteinase K, 10mM Kaliumacetat, 10 mM HEPES, 150 mM NaCl, pH 4.2). Nach der Hydratisierung des Films werden die gebildeten Liposomen durch eine 400 nm-Membran extrudiert. Nicht eingeschlossene Proteinase wird durch Flotation der Liposomen im Sucrosegradienten abgetrennt.

Die so hergestellten Liposomen werden mit 7.5 ml Puffer bei pH 4.2 und pH 7.2 inkubiert (Puffer wie oben, Ausgangs-pH 4.2 und 8.0). Nach der Inkubation wird freigesetzte Proteinase K durch Ultrafiltration mit einer 0.1 $\mu$m-Membran abgetrennt. Die im Filter verbleibenden Liposomen werden dann mit 7.5 ml einer Lösung von Triton X-100 in Puffer (wie oben, pH 8.0) behandelt.

Alle Filtrate werden auf die Anwesenheit von Proteinase K getestet. Dazu wird eine Lösung von Azocasein (6 mg/ml Azocasein in 1M Harnstoff, 200 mM Tris-Sulfat pH 8.5)verwendet. 500$\mu$l dieser Lösung werden mit 100 $\mu$l Filtrat oder Puffer gemischt und für 30min bei 37°C inkubiert. Die Reaktion wird durch Zugabe von 10% Trichloressigsäure gestoppt. Präzipitierte Proteine werden durch Zentrifugation abgetrennt. Die Färbung im Überstand wird bei 390 nm gemessen.

| pH Inkubation | Triton X100 | Absorption bei 390nm - Blank |
|---|---|---|
| 4.2 | - | 0,0165 |
| 4.2 | + | 0,1731 |
| 7.2 | - | 0,1354 |
| 7.2 | + | 0,0260 |

Erfolgt die Inkubation der Liposomen bei einem pH-Wert von 4.2, so wird keine oder nur sehr wenig Proteinase K freigesetzt. Erst die Auflösung der Liposomen mit Triton X100 führt zur Freisetzung des Enzyms.

Wenn die Liposomen bei einem pH-Wert von 7.2 inkubiert werden, so wird bereits ohne Zugabe von Triton der Großteil des Enzyms freigesetzt und findet sich im ersten Filtrat. Die Zugabe von Triton kann dann kaum noch weiteres Enzym aus den Liposomen herauslösen.

**Beispiel 10**

Zytotoxizität

Die toxische Wirkung der Substanzen wurde mit dem MTT-Test untersucht. Dazu wurden HeLa-Zellen mit einer Dichte von $2*10^4$ Zellen pro Kavität einer 96-well-Titerplatte ausgesät und für zwei Tage kultiviert. Liposomen verschiedener Zusammensetzung (s. Tabelle) wurden in einer Konzentration von 0,5 mM zu den Zellen gegeben und 24h mit diesen Zellen inkubiert. Anschließend wurde der MTT-Test durchgeführt.

| Liposom | viability (MTT) | Bemerkungen |
|---|---|---|
| - | 100% | |
| DOPE 60/ DOTAP 40 | 82% | |
| DOPE 60/ DC-Chol 40 | 57% | Ablösung der Zellen |
| DOPE 60/ His-Chol 40 | 92% | |
| DOPE 60/ Mo-Chol 40 | 103% | |
| POPC 60/ His Chol 40 | 90% | |
| DOPE: Dioleoylphosphatidylethanolamin, jeweils 60mol% | | |
| POPC: Palmitoyloleoylphsophatidylcholin, jeweils 60mol% | | |
| DC-Chol: N,N-Dimethyl(2-aminoethyl)carbamoylcholesterol | | |

Auch in hohen Konzentrationen werden Liposomen, umfassend die erfindungsgemäßen Substanzen , sehr gut von Zellen vertragen. Eine Toxische Wirkung ist kaum nachzuweisen. Im Gegensatz dazu besitzen bekannte kationische Lipide wie DC-Chol oder DOTAP eine signifikante zytotoxische Wirkung.

**Beispiel 11**

Stabilität in Serum

Carboxyfluorescein beladene Liposomen der Zusammensetzungen POPC/His-Chol 60:40, POPC/Mo-Chol 60:40 und POPC/DPPG 60:40 (alle Angaben in mol%)wurden analog Beispiel 5 hergestellt. Zur Messung wurden die Liposomen auf 0.1 mM in Humanserum verdünnt und bei 37°C inkubiert. Zu bestimmten Zeitpunkten wurde die Fluoreszenz gemessen. Vollständige Freisetzung von CF wurde durch Zusatz von Triton X-100 zum Messpuffer erreicht. Die gewonnen CF-Freisetzungsdaten sind in der untenstehenden Tabelle zusammengestellt. Zum Vergleich sind die negativ geladenen POPC/DPPG-Liposomen dargestellt, die im Verlauf von 4 h praktisch kein CF verlieren. POPC/His-Chol-Liposomen zeigen bis 2 h sehr gute Serumstabilitäten, verlieren aber bis 4 h etwas CF. POPC/Mo-Chol-Liposomen zeigen etwas mehr Permeabilität als POPC/His-Chol.

| | POPC/His-Chol | POPC/Mo-Chol | POPC/DPPG |
|---|---|---|---|
| 0 min | 0 % | 0 % | 0 % |
| 30 min | 0 % | 4 % | 0 % |
| 60 min | 2 % | 5 % | 1 % |
| 120 min | 4 % | 9 % | 2 % |
| 240 min | 19 % | 16 % | 2 % |

**Beispiel 12**

Transfektion in Zellen

HeLa-Zellen ($3*10^5$) wurden in jede Kavität einer 6-well Titerplatte ausplattiert und für drei Tage kultviert.

Liposomen wurden mit gleichen Zusammensetzungen wie in Beispiel 10 in Gegenwart von fluoreszensmarkiertem Dextran (TRITC-Dextran, 10 mg/ml im Hydratisierungspuffer) hergestellt.Nicht eingebautes TRITC-Dextran wurde durch Gelfiltration entfernt. Die so hergestellten Liposomen wurden zu den Zellen gegeben und für 6h bei 37°C inkubiert. Anschließend wurden die Zellen zweimal mit Puffer gewaschen. Die Aufnahme des Dextrans wurde im mikroskopischen Bild verfolgt bzw. mittels Fluoreszensspektroskopie quantifiziert.

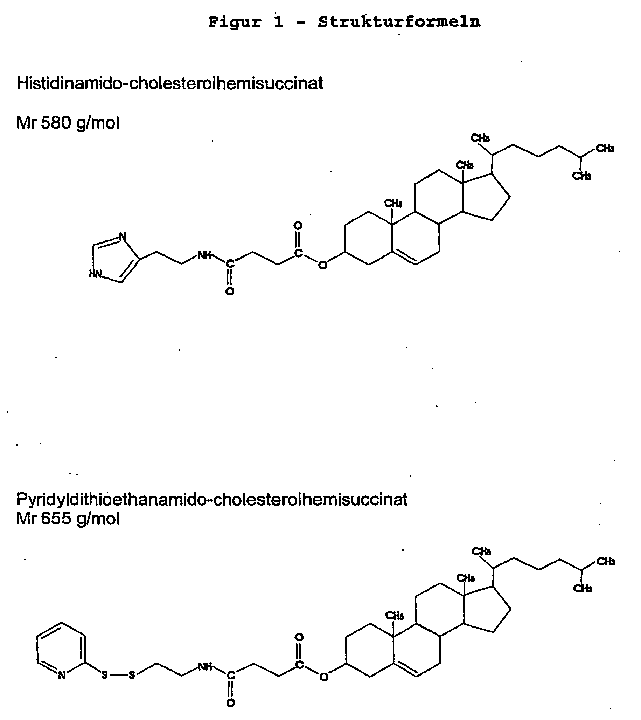

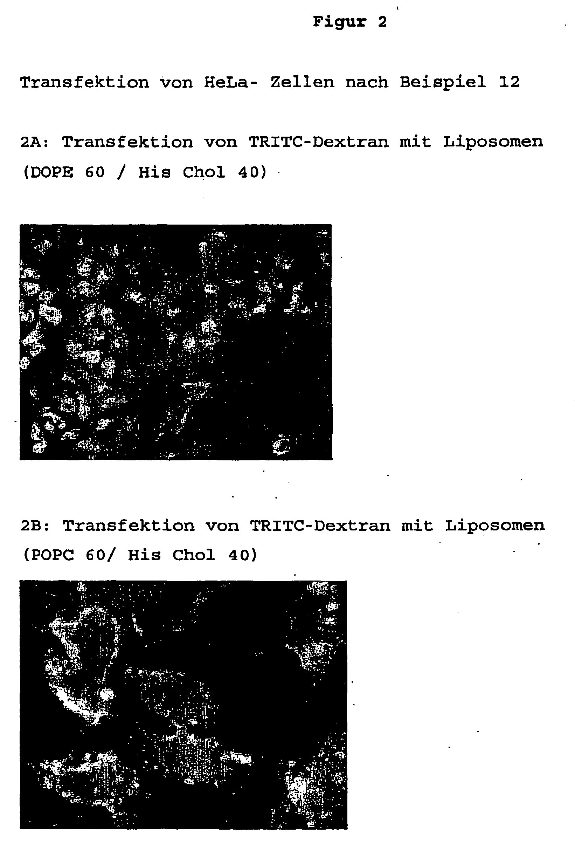

Die Ergebnisse sind in der untenstehenden Tabelle und in Figur 2 zusammengefasst.

| Liposom | aufgenommenes TRITC-Dextran in $\mu$g |
|---|---|
| - (TRITC-Dextran in Puffer) | 0,1 |
| DOPE 60 / DOTAP 40 | 1,5 |
| DOPE 60 / DC-Chol 40 | 1,1 |
| DOPE 60 / His Chol 40 | 0,3 |
| DOPE 60 / Mo Chol 40 | 0,7 |
| POPC 60 / His Chol 40 | 0,4 |

Die neuen Verbindungen erreichen hier noch nicht die Effizienz der bekannten kationischen Lipide DOTAP oder DC-Chol. Allerdings vermitteln auch sie die Transfektion von Makromolekülen in Zellen. Es ist zu erwarten, dass sich diese Effizienz durch Verwendung von Liganden zur Zellanheftung noch erheblich steigern lässt.

**Patentansprüche**

1. Sterolderivat nach der allgemeinen Formel (1):

$$\text{Kation} - \text{Spacer 2} - Y - \text{Spacer 1} - X - \text{Sterol} \qquad (1),$$

wobei

- das Kation eine Stickstoffbase ist ausgewählt aus der Gruppe enthaltend Piperazine, Imidazole, Morpholine, Purine, Pyrimidinen und 2-, 3- oder 4- modifizierte Pyridine, die weitere Substituenten aufweisen können.
- die Spacer 1 und 2 Niederalkylreste mit bis zu 8 C-Atomen mit linearer, verzweigter oder ringförmiger Struktur und 0, 1 oder 2 ethylenisch ungesättigten Bindungen sind und Hydroxylgruppen besitzen können,
- die verbindenden Gruppen X und Y ausgewählt sind aus der Gruppe bestehend aus -(C=O)-O-; -(C=O)-NH-; -(C=O)-S-; -O-; -NH-; -S-; -CH=N-; -O-(O=C)-; -S-(O=C)-; -NH-(D=C)- und -N=CH-,.
- das Sterol ausgewählt ist aus der Gruppe bestehend aus Cholesterol, Sitosterol, Campesterol, Desmosterol, Fucosterol, 22-Ketosterol, 20-Hydroxysterol, Stigmasterol, 22-Hydroxycholesterol, 25-Hydroxycholesterol, Lanosterol, 7-Dehydrocholesterol, Dihydrocholesterol, 19-Hydroxycholesterol, 5$\alpha$-Cholest-7-en-3$\beta$-ol, 7-Hydroxycholesterol, Epicholesterol, Ergosterol und/oder Dehydroergosterol und
- das Gesamtmolekül einen pKa-Wert zwischen 3,5 und 8 aufweist.

2. Sterolderivat nach Anspruch 1, **dadurch gekennzeichnet, dass** das Sterolderivat einen pKa-Wert zwischen 4 und 6,5 aufweist.

3. Liposomen umfassend Sterolderivate nach einem der Ansprüche 1 bis 2.

4. Liposomen nach dem vorhergehenden Anspruch, **dadurch gekennzeichnet, dass** die Liposomen zwischen 5 mol% und 50 mol%, bevorzugt zwischen 5 mol% und 40 mol%, besonders bevorzugt zwischen 10 mol% und 30 mol% Sterolderivate umfassen.

5. Liposomen nach Anspruch 3 oder 4, **dadurch gekennzeichnet, dass** die Liposomen Phosphatidylcholin, Phosphatidylethanolamin und/oder Diacylglycerol umfassen.

6. Liposomen nach einem der Ansprüche 3 bis 5, **dadurch gekennzeichnet, dass** die Liposomen eine mittlere Größe zwischen 50 und 1000 nm, bevorzugt 50 und 300 nm, besonders bevorzugt 60 und 130 nm aufweisen.

7. Liposomen nach einem der Ansprüche 3 bis 6, **dadurch gekennzeichnet, dass** die Liposomen einen Wirkstoff umfassen.

8. Liposomen nach dem vorhergehenden Anspruch, **dadurch gekennzeichnet, dass** der Wirkstoff ein Protein, ein Peptid, eine DNA, eine RNA, ein antisense-Nukleotid und/oder ein Decoy-Nukleotid ist.

9. Liposomen nach einem der Ansprüche 6 bis 8, **dadurch gekennzeichnet, dass** sich mindestens 80% des Wirkstoffes im Innern des Liposoms befinden.

10. Verfahren zur Wirkstoffbeladung von Liposomen nach einem der Ansprüche 3 bis 9, **dadurch gekennzeichnet, dass** ein definierter pH-Wert zur Verkapselung benutzt wird und ein zweiter pH-Wert zur Abtrennung des nicht gebundenen Wirkstoffes eingestellt wird.

11. Verfahren zur Wirkstoffbeladung von Liposomen nach einem der Ansprüche 3 bis 9, **dadurch gekennzeichnet, dass** die Liposomen bei einem definierten pH-Wert permeabilisiert und folgend verschlossen werden.

12. Verwendung von Liposomen nach einem der Ansprüche 3 bis 9 zur Herstellung von Nanokapseln.

13. Verwendung von Liposomen nach einem der Ansprüche 3 bis 9 zur Herstellung von Freisetzungssystemen in der Diagnostik.

14. Liposomen nach einem der Ansprüche 3 bis 9 zum Transport und/oder zur Freisetzung von Wirkstoffen.

15. Liposomen nach einem der Ansprüche 3 bis 9 zur Verwendung als Depotformulierung und/oder als zirkulierendes Depot.

16. Liposomen nach einem der Ansprüche 3 bis 9 zur Verwendung bei intravenöser oder peritonealer Applikation.

17. Verwendung von Liposomen nach einem der Ansprüche 3 bis 9 als Vektor zur Transfektion von Zellen in vitro und ex vivo.

18. Liposomen nach einem der Ansprüche 3 bis 9, zur Verwendung als Vektor zur Transfektion von Zellen in vivo.

**Claims**

1. A sterol derivative according to general formula (1):

$$\text{cation - spacer 2 - Y - spacer 1 - X - sterol} \quad (1),$$

wherein

- the cation is a nitrogen base selected from the group consisting of piperazines, imidazoles, morpholines, purines, pyrimidines and 2-, 3- or 4- modified pyridines, which can have further substituents,
- the spacers 1 and 2 are lower alkyl residues with up to 8 C atoms with a linear, branched or cyclic structure and 0, 1 or 2 ethylenically unsaturated bonds and can possess hydroxyl groups,
- the linking groups X and Y are selected from the group consisting of
- (C=O)-O-, -(C=O)-NH-, -(C=O)-S-, -O-, -NH-, -S-, -CH=N-,
- O-(O=C)-, -S-(O=C)-, -NH-(O=C)-, and -N=CH-,
- the sterol is selected from the group consisting of cholesterol, sitosterol, campesterol, desmosterol, fucosterol, 22-ketosterol, 20-hydroxysterol, stigmasterol, 22-hydroxycholesterol, 25-hydroxycholesterol, lanosterol, 7-dehydrocholesterol, dihydrocholesterol, 19-hydroxycholesterol, $5\alpha$-cholest-7-en-3$\beta$-ol, 7-hydroxycholesterol, epicholesterol, ergosterol, and/or dehydroergosterol,
- the overall molecule has a pKa value of between 3.5 and 8.

2. The sterol derivative according to claim 1, **characterized in that** the sterol derivative has a pKa value of between 4 and 6,5.

3. Liposomes comprising the sterol derivatives according to any of claims 1 to 2.

4. The liposomes according to the preceding claim, **characterized in that** the liposomes comprise between 5 mole-% and 50 mole-%, preferably between 5 mole-% and 40 mole-%, more preferably between 10 mole-% and 30 mole-% of sterol derivatives.

5. The liposomes according to claim 3 or 4, **characterized in that** the liposomes comprise phosphatidyl choline, phosphatidyl ethanolamine and/or diacylglycerol.

6. The liposomes according to any of claims 3 to 5, **characterized in that** the liposomes have an average size of between 50 and 1000 nm, preferably between 50 and 300 nm, and more preferably between 60 and 130 nm.

7. The liposomes according to any of claims 3 to 6, **characterized in that** the liposomes comprise an active substance.

8. The liposomes according to the preceding claim, **characterized in that** the active substance is a protein, a peptide, a DNA, an RNA, an antisense nucleotide and/or a decoy nucleotide.

9. The liposomes according to any of claims 6 to 8, **characterized in that** at least 80% of the active substance is situated inside the liposome.

10. A method of loading the liposomes according to any of claims 3 to 9 with active substance, **characterized in that** one defined pH value is used for encapsulation, and a second pH value is adjusted for removal of unbound active substance.

11. A method of loading the liposomes according to any of claims 3 to 9 with active substance, **characterized in that** the liposomes are made permeable at a defined pH value and subsequently sealed.

12. Use of the liposomes according to any of claims 3 to 9 in the production of nanocapsules.

13. Use of the liposomes according to any of claims 3 to 9 in the production of release systems in diagnostics.

14. Liposomes according to any of claims 3 to 9 for the transport and/or release of active substances.

15. Liposomes according to any of claims 3 to 9 for the use as depot formulation and/or as circulative depot.

16. Liposomes according to any of claims 3 to 9 for the use in intravenous or peritoneal application.

17. Use of the liposomes according to any of claims 3 to 9 as vector to transfect cells *in vitro* and *ex vivo*.

18. Liposoemes according to any of claims 3 to 9, for the use as vector to transfect cells *in vivo*.

**Revendications**

1. Dérivé de stérol selon la formule générale (1) :

$$\text{cation} - \text{espaceur 2} - \text{Y} - \text{espaceur 1} - \text{X} - \text{stérol} \qquad (1)$$

dans laquelle

- le cation est une base azotée choisie parmi le groupe comprenant les pipérazines, les imidazoles, les morpholines, les purines, les pyrimidines et les pyridines 2-, 3- ou 4-modifiées, qui peuvent comporter d'autres substituants,
- les espaceurs 1 et 2 sont des radicaux alkyles inférieurs comportant jusqu'à 8 atomes de carbone, ayant une structure linéaire, ramifiée ou cyclique et 0, 1 ou 2 liaisons éthyléniquement insaturées et peuvent comporter des groupes hydroxyle,

- les groupes de liaison X et Y sont choisis parmi le groupe comprenant -(C=O)-O-, -(C=O)-NH-, -(C=O)-S-, -O-, -NH-, -S-, -CH=N-, -O-(O=C)-, -S-(O=C)-, -NH-(O=C)- et -N=CH-,

- le stérol est choisi parmi le groupe comprenant le cholestérol, le sitostérol, le campestérol, le desmostérol, le fucostérol, le 22-cétostérol, le 20-hydroxystérol, le stigmastérol, le 22-hydroxycholestérol, le 25-hydroxycholestérol, le lanostérol, le 7-déhydrocholestérol, le dihydrocholestérol, le 19-hydroxycholestérol, le 5$\alpha$-cholest-7-én-3$\beta$-ol, le 7-hydroxycholestérol, l'épicholestérol, l'ergostérol et/ou le déhydroergostérol et

- la molécule entière a un pKa entre 3,5 et 8.

2. Dérivé de stérol selon la revendication 1, **caractérisé en ce que** le dérivé de stérol a un pKa entre 4 et 6,5.

3. Liposomes comprenant des dérivés de stérol selon l'une des revendications 1 à 2.

4. Liposomes selon la revendication précédente, **caractérisés en ce que** les liposomes comprennent entre 5 % en moles et 50 % en moles, de préférence entre 5 % en moles et 40 % en moles, et de manière encore plus préférée entre 10 % en moles et 30 % en moles de dérivés de stérol.

5. Liposomes selon la revendication 3 ou 4, **caractérisés en ce que** les liposomes comprennent de la phosphatidylcholine, de la phosphatidyléthanolamine et/ou du diacylglycérol.

6. Liposomes selon l'une des revendications 3 à 5, **caractérisés en ce que** les liposomes ont une dimension moyenne entre 50 et 1000 nm, de préférence entre 50 et 300 nm, et de manière encore plus préférée entre 60 et 130 nm.

7. Liposomes selon l'une des revendications 3 à 6, **caractérisés en ce que** les liposomes contiennent un principe actif.

8. Liposomes selon la revendication précédente, **caractérisés en ce que** le principe actif est une protéine, un peptide, un ADN, un ARN, un nucléotide antisens et/ou un nucléotide leurre.

9. Liposomes selon l'une des revendications 6 à 8, **caractérisés en ce qu'**au moins 80 % du principe actif se trouve à l'intérieur du liposome.

10. Procédé de chargement en principe actif des liposomes selon l'une des revendications 3 à 9, **caractérisé en ce qu'**on utilise un pH défini pour l'encapsulage et on règle un second pH pour séparer le principe actif non lié.

11. Procédé de chargement en principe actif des liposomes selon l'une des revendications 3 à 9, **caractérisé en ce qu'**on perméabilise les liposomes à un pH défini et qu'on les obture ensuite.

12. Utilisation de liposomes selon l'une des revendications 3 à 9 pour préparer des nanocapsules.

13. Utilisation de liposomes selon l'une des revendications 3 à 9 pour préparer des systèmes de libération dans l'art du diagnostic.

14. Liposomes selon l'une des revendications 3 à 9 pour le transport et/ou la libération de principes actifs.

15. Liposomes selon l'une des revendications 3 à 9 destinés à une utilisation en tant que formulation retard et/ou formule retard circulant.

16. Liposomes selon l'une des revendications 3 à 9 destinés à une utilisation pour une application par voie intraveineuse ou péritonéale.

17. Utilisation de liposomes selon l'une des revendications 3 à 9 en tant que vecteur de transfection de cellules in vitro et ex vivo.

18. Liposomes selon l'une des revendications 3 à 9 destinés à une utilisation en tant que vecteur de transfection de cellules in vivo.

## Figur 1 - Strukturformeln

Histidinamido-cholesterolhemisuccinat

Mr 580 g/mol

Pyridyldithioethanamido-cholesterolhemisuccinat
Mr 655 g/mol

**Figur 2**

Transfektion von HeLa- Zellen nach Beispiel 12

2A: Transfektion von TRITC-Dextran mit Liposomen (DOPE 60 / His Chol 40)

2B: Transfektion von TRITC-Dextran mit Liposomen (POPC 60/ His Chol 40)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4891208 A
- WO 0028972 A
- WO 0164330 A

### In der Beschreibung aufgeführte Nicht-Patentliteratur

- **TACHIBANA et al.** *BBRC,* 1998, vol. 251, 538-544
- *Nature Biotechnology,* 1996, vol. 14, S.760-7
- Handbook of Chemistry and Physics. vol. 73, 8-37 ff